# EUROPEAN PATENT SPECIFICATION

(11) **EP 1 955 334 B1**
(45) Date of publication and mention of the grant of the patent: **11.04.2012**
(21) Application number: 06807977.1
(22) Date of filing: 01.11.2006
(51) Int. Cl.: G21F 3/00, A61B 6/10

(54) **RADIATION SHIELD**
STRAHLUNGSSCHIRM
ECRAN ANTI-RADIATION

(30) Priority: 02.11.2005 FI 20050358 U
(43) Date of publication of application: 13.08.2008
(73) Proprietor: Ahonen, Päivi, 80160 Joensuu (FI); Räsänen, Outi, 80170 Joensuu (FI)
(72) Inventor: Ahonen, Päivi, 80160 Joensuu (FI); Räsänen, Outi, 80170 Joensuu (FI)
(74) Representative: Pitkänen, Hannu Alpo Antero
(86) International application number: PCT/FI2006/000350
(87) International publication number: WO 2007/051899

(56) References cited:
- WO-A1-01/26551
- WO-A1-01/26551
- WO-A2-2005/094272
- DE-A1- 1 466 848
- DE-U1- 29 706 321
- US-A- 4 938 233
- US-A- 5 012 114
- US-A- 5 417 225
- US-A1- 2005 213 712

## Description

The present invention relates to a radiation shield used in X-ray imaging or other medical procedures involving a radiation hazard.

Radiation shields are used in medical procedures, for example for protecting the staff members operating X-ray equipment as well as patients from detrimental stray radiation. It is known that X-ray examinations are used extensively in hospitals and in other medical procedures and that patients and staff are exposed to hazardous X-ray radiation. According to the general principles of radiation protection, radiation exposure detrimental to the health of the patient should be kept as low as practicable. X-ray radiation carries enough energy to cause changes in living tissue. Normally, such changes are self-repairing and thus insignificant. A very low percentage of the changes may, however, cause defects in the genetic code of a cell that may lead to the generation of cancer cells. The X-ray radiation targeted at a patient propagates as what is known as scattered radiation (stray radiation) throughout the examination room. Particularly in an operating theatre, the staff cannot move away from the patient. In X-ray departments, radiation shields fixed to the rails of the imaging table are used to reduce stray radiation. Such shields are not, however, suitable for use on operating tables, hospital beds or narrow procedure tables used for manual surgical procedures because they lack a solid metal rail to which the radiation shield could be mounted. The shields currently available (such as vests, vest-and-skirt combinations, thyroid shields) consist of personal protective gear that does not protect the limbs, head, etc., from stray radiation. Portable wall shields are heavy and take up a lot of space. Working with such shields on a continuous basis in close contact with the patient is impossible. At the same time, protecting the other patients in the same room is quite complicated because of the cramped space.

US patents 6,481,888 B1 (Morgan 2002), 5,006,718 (Lennart 1991) and 5,9000,638 (Siemens AG 1999) and German patents DE 297 06 321 U1 (1997) and DE 103 25 567 A1 (2005) provide descriptions of equipment designed for protection against X-ray radiation. Strip-like or board-like in structure, these shields allow the use of a portable X-ray tube. Because of the fixing system used, these devices cannot, however, be mounted onto all types of procedure or operating tables (of different width, length and/or height) and/or hospital beds. These shields are designed for specific procedures and tables and cannot thus be adapted to a range of procedures or needs.

Document WO 01/26551 disclosed a drape composed of an X-ray permeable fabric. Various X-shielding panels can be appended to the drape by employing attachment means.

The purpose of the present invention is to provide a radiation shield that eliminates a number of the drawbacks associated with the existing shields. More specifically, the invention seeks to provide a radiation shield that can be adapted to different conditions, procedures, and needs. A further objective of the invention is to provide a radiation shield that improves the standard of radiation protection for the staff and patients.

The purpose of the invention is achieved with a radiation shield, the characteristics of which are presented in the claims.

In the radiation shield in accordance with the invention, hook and loop fasteners are attached to a side of the radiation-permeable zone section, the radiation-permeable zone is to be placed on the procedure table extending from other one side of the protective table to the opposite side and, that the radiation shield is formed by attaching the two or several separate parts of the radiation shield to each other by placing the radiation-permeable zones sections on top of each other so, that the sides with hook and loop fasteners are against each other and are arranged to fix separate parts to one another non-permanently. With the hook and look fasteners, as so-called Velcro fasteners, (two or more) parts of the radiation shield can be simply and easily fixed to each other in the preferred way and at the preferred points in order to create a radiation shield of desired shape and size. The Velcro fasteners can be overlap wherever desired, allowing the user to lengthen or shorten the shield. In other circumstances, the Velcro fasteners can be placed crosswise relative to one another, or on top of one another parallel to the shield. As a result, the radiation shield in accordance with the invention can be used on hospital beds, procedure tables, operating tables and stands of different type and size. This kind of radiation shield is easy to mount to structures of different size and shape. Additionally, it takes up little space in storage.

In a preferred embodiment of the invention, the Velcro fasteners are attached to both sides of the radiation-permeable zone of a part of the shield. These are essentially made of thinner material that the radiation-impermeable or radiation-absorbing zone, making it easy to attach the parts to one another in a number of ways.

In another preferred embodiment of the invention, the protective radiation-impermeable or radiation-absorbing zone of the shield consists of partially overlapping strip-like parts attached to the radiation-permeable zone. The overlapping strip-like parts provide efficient protection against scattered radiation. Typical of the radiation shield in accordance with the invention is that it consists of two or several parts and can be linked at the preferred points using Velcro fasteners. As a rule, the basic shield consists of two parts to which additional parts of different sizes can be attached using Velcro fasteners. Previously, protecting other patients around the patient being X-rayed required complicated arrangements because there is a limited number of protective screens available in the ward or they are not practicable because of the cramped space; however, the other patients can be effectively protected using this type of radiation shield. With the radiation shield in accordance with the present invention, stray radiation around the patient can be reduced. The shield is easy to install and move from one bed to another. The other advantages offered by the invention is that it is light in weight, easy to move and makes it possible to use a portable X-ray unit. Until now no radiation shields have been commercially available that would reduce stray radiation and allow the use of a portable X-ray unit. A radiation shield in accordance with the present invention improves the standard of radiation protection for the staff as well as for the patients.

The protective radiation-impermeable or radiation-absorbing zone of the radiation shield includes strips of rubber and lead that make it possible to move the X-ray unit in different directions (e.g. for the purpose of vertical or horizontal imaging) while at the same time reducing hazardous stray radiation. Additionally, the radiation shield is easy to clean compared with rail-mounted shields because of the large number of notches and cavities they include.

In the following, the invention will be explained in more detail with reference to the attached drawings, in which
Figure 1 illustrates a top view of a radiation shield in accordance with the invention; and
Figure 2 illustrates an oblique top view of another radiation shield in accordance with the invention while illustrating the way it is used.

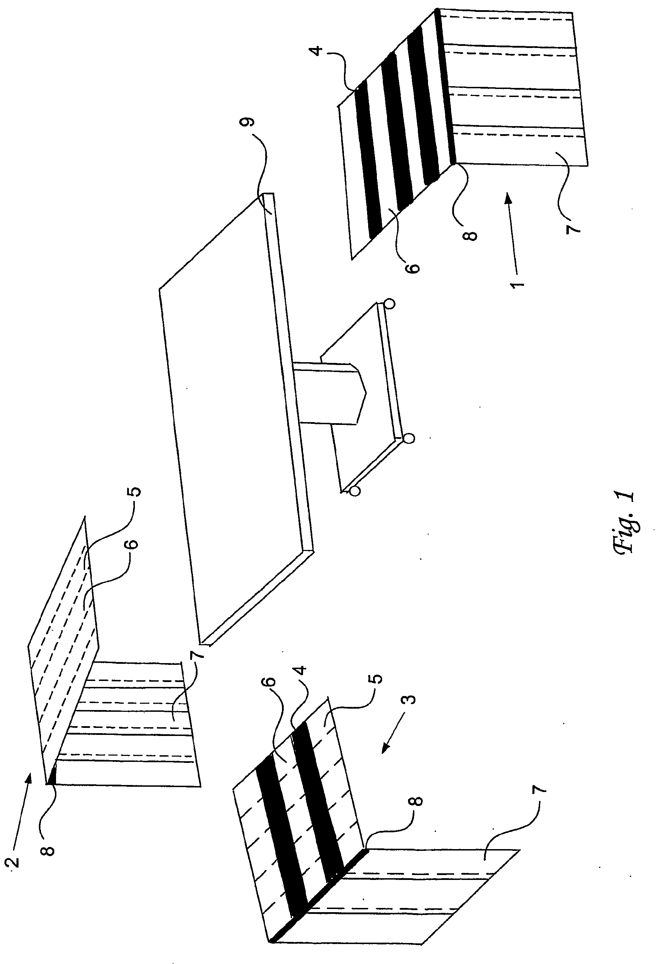

The embodiment shown in Figure 1 consists of two separate parts, 1 and 2. Part 1 is fitted with Velcro fasteners 4 and part 2 with Velcro fasteners 5. The Velcro fasteners 4 are hooks and the Velcro fasteners 5 are loops that form a firm bond when pressed together.

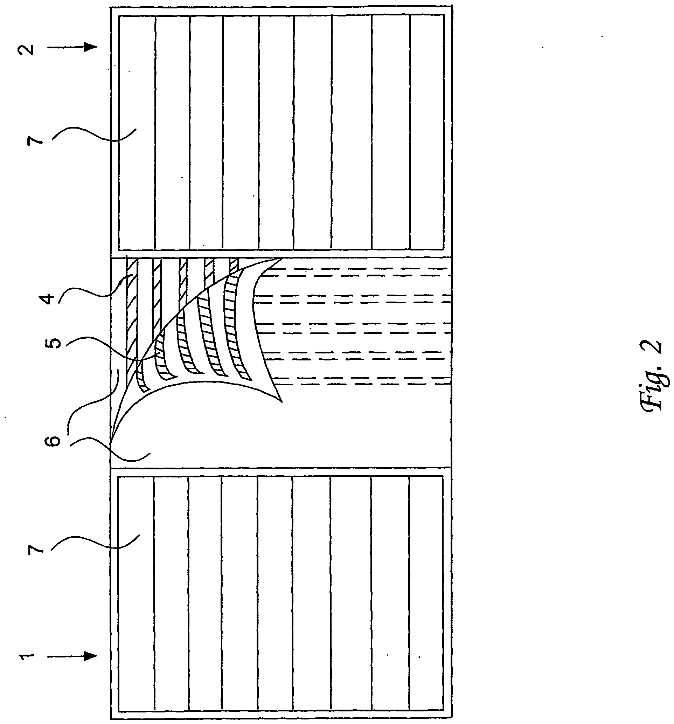

The embodiment shown in Figure 2 consists of three separate parts, 1, 2 and 3. Part 1 is the lowermost base section of the radiation shield fitted with Velcro fasteners 4. Part 2 is the uppermost section of the radiation shield fitted with Velcro fasteners 5. Velcro fasteners 4 are loops and Velcro fasteners 5 are hooks ensuring a strong bond between the two. Part 3 is an additional section in between said sections provided with Velcro fasteners on both sides.

Parts 1, 2, and 3 of the radiation shield presented in the Figures include the radiation-permeable zone 6 as well as the protective radiation-impermeable or radiation-absorbing zone 7. In between these two zones there is the strip attachment joint 8 that can be hot-sealed to parts made of plastic. Velcro fasteners 4 and 5 are attached to one side of the radiation-permeable zone 6 either transversely (Figure 1 and Figure 2, part 1) or longitudinally (Figure 1 and Figure 2, part 2), or to both sides (Figure 2, part 3). The protective radiation-impermeable or radiation-absorbing zone of parts 1, 2, and 3 of the radiation shield consists, in these embodiments, of partly overlapping strip-like sections that are attached to the radiation-permeable zone 6. The strip-like sections are made of rubber and lead coated with a protective material.

As shown in Figure 1, parts 1 and 2 of the radiation shield are attached to each other by placing the radiation-permeable zones on top of each other in such a way that the Velcro fasteners are crosswise. In the embodiment in accordance with Figure 2, the radiation-permeable zones of parts 1, 2, and 3 are placed on top of each other on the procedure table 9 in said order, meaning that part 1 is uppermost, part 2 in the middle, and part 3 lowermost. The individual parts are attached to one another with Velcro fasteners in the manner explained above. The protective radiation-impermeable or radiation-absorbing zones 7 are placed on the individual sides of the procedure table and suspended from it.

In one embodiment of the invention, the radiation shield includes a strip-like shield attached to the rail fixed to the edge of the procedure table or hospital bed. As already explained, the shield includes the preferred number of protective strips that are attached (for example by hot-sealing) to a low base section featuring hook fasteners, to which the preferred number of narrow strips with loop fasteners are attached that can be used for suspending the shield from the edge of the procedure table. The fastener loops make it possible to use the shield on different tables irrespective of the edge rail system they have. The advantage offered by the shield is that if the (original) shield presented earlier has not been placed in position on the procedure table (under the patient) and it cannot be placed there afterwards because of the poor condition of the patient (e.g. multiple injuries), this suspension model serves as an equivalent protective shield against stray radiation that the patient and staff would otherwise be exposed to. At the same time, the shield permits the use of a mobile X-ray unit as already explained.

In other examples, the numbers of parts may vary as required. Additionally, the parts can be attached to one another either crosswise or lengthwise on top of one another as preferred. Consequently, the Velcro fasteners can be laid longitudinally, transversely or obliquely relative to the parts. Additionally, the lengths and widths of the Velcro fasteners can vary from one application to another. In some of them, it is possible to use bits of a Velcro fastener attached to the part as appropriate. The Velcro fasteners can be placed crosswise relative to one another or lengthwise on top of one another.

The advantageous applications of the invention are not limited to the above examples; instead, the invention and its embodiments may be varied within the scope of protection provided by the claims.

## Claims

1. A radiation shield used in x-ray imaging or other medical procedures involving a radiation hazard, which is made of a flexible material consisting of two or several parts (1, 2, 3) with a radiation-permeable zone (6) and a radiation-absorbing zone (7) and one or several hook and loop fasteners (4, 5) for fixing the radiation-permeable zone (6) and the radiation-absorbing protective zone (7) to one another, ***characterized* in, that** the hook and loop fasteners (4, 5) are attached to a side of the radiation-permeable zone (6), the radiation-permeable zone (6) is to be placed on the procedure table extending from other one side of the protective table to the opposite side and, that the radiation shield is formed by attaching the two or several separate parts (1,2,3) of the radiation shield to each other by placing the radiation-permeable zones (6) on top of each other so, that the hook and loop fasteners (4, 5) fix the two or several separate parts (1, 2, 3) to one another non-permanently.

2. A radiation shield in accordance with claim 1, ***characterized* in, that** hook and loop fasteners (4, 5) are attached to both sides of the radiation-permeable zone (6).

3. A radiation shield in accordance with claim 1 or 2, ***characterized* in, that** the protective radiation-impermeable or radiation-absorbing zone (7) of the shield consists of partially overlapping strip-like parts attached to the radiation-permeable zone (6).

## Patentansprüche

1. Strahlungsschutzschirm, welcher bei der Röntgenbildgebung oder anderen medizinischen Verfahren, bei denen eine Strahlungsgefahr vorhanden ist, verwendet wird, welcher aus einem flexiblen Material hergestellt ist und aus zwei oder mehreren Teilen (1, 2, 3) mit einem strahlungsdurchlässigen Bereich (6) und einem strahlungsabsorbierendem Bereich (7) und einem oder mehreren Klettverschlüssen (4, 5) zum Befestigen des strahlungsdurchlässigen Bereiches (6) und des strahlungsabsorbierenden Schutzbereiches (7) aneinander besteht, **dadurch *gekennzeichnet,* dass** die Klettverschlüsse (4, 5) an einer Seite des strahlungsdurchlässigen Bereiches (6) angebracht sind, der strahlungsdurchlässige Bereich (6) auf dem Behandlungstisch anzuordnen ist, so dass er sich von der einen Seite des Schutztisches bis zur gegenüberliegenden Seite erstreckt, und dass der Strahlungsschutzschirm gebildet wird, indem die zwei oder mehreren getrennten Teile (1, 2, 3) des Strahlungsschutzschirms aneinander befestigt werden, indem die strahlungsdurchlässigen Bereiche (6) übereinander angeordnet werden, so dass die Klettverschlüsse (4, 5) die zwei oder mehreren getrennten Teile (1, 2, 3) lösbar aneinander befestigen.

2. Strahlungsschutzschirm nach Anspruch 1, **dadurch *gekennzeichnet,* dass** die Klettverschlüsse (4, 5) an beiden Seiten des strahlungsdurchlässigen Bereiches (6) angebracht sind.

3. Strahlungsschutzschirm nach Anspruch 1 oder 2, **dadurch *gekennzeichnet,* dass** der strahlungsundurchlässige oder strahlungsabsorbierende Schutzbereich (7) des Schutzschirms aus sich teilweise überlappenden streifenartigen Teilen besteht, die an dem strahlungsdurchlässigen Bereich (6) angebracht sind.

## Revendications

1. Ecran anti-radiations utilisé dans l'imagerie par rayons x ou autre procédures médicales impliquant un risque de radiation, qui est fait d'un matériau flexible consistant en deux ou plusieurs parties (1, 2, 3) avec une zone perméable aux radiations (6) et une zone absorbant les radiations (7) et une ou plusieurs fixation(s) à boucle et crochet (4, 5) pour fixer la zone perméable aux radiations (6) et la zone protectrice absorbant les radiations (7) l'une à l'autre, **caractérisé en ce que** les fixations à boucle et crochet (4, 5) sont fixées sur un côté de la zone perméable aux radiations (6), la zone perméable aux radiations (6) devant être placée sur la table de procédure s'étendant depuis l'un côté de la table protectrice vers l'autre, et **en ce que** l'écran anti-radiations est formé en fixant les deux ou plusieurs pièces séparées (1, 2, 3) de l'écran anti-radiations l'une à l'autre en plaçant les zones perméables aux radiations (6) l'une au-dessus de l'autre de façon que les deux ou plusieurs fixations à boucle et crochet (4, 5) fixent les deux ou plusieurs pièces séparées (1, 2, 3) l'une à l'autre de façon non-permanente.

2. Ecran anti-radiations selon la revendication 1, **caractérisé en ce que** les fixations à boucle et crochet (4, 5) sont fixées sur les deux côtés de la zone perméable aux radiations (6).

3. Ecran anti-radiations selon la revendication 1 ou 2, **caractérisé en ce que** la zone protectrice imperméable aux radiations ou absorbant les radiations (7) de l'écran consiste en des parties semblables à des bandes se chevauchant partiellement, fixées sur la zone perméable aux radiations (6).
